(19) Europäisches Patentamt
European Patent Office
Office européen des brevets

(11) Publication number: 0 004 430
B1

(12) EUROPEAN PATENT SPECIFICATION

(45) Date of publication of patent specification: 23.12.81

(51) Int. Cl.[3]: C 07 J 9/00

(21) Application number: 79300366.6

(22) Date of filing: 09.03.79

(54) A process for the selective conversion of 20-methylpregna-3,5,20(21)-triene-3,21-diol-diacetate to 3-oxo-20-methyl-pregna-4,20(21)-diene-21-yl-acetate.

(30) Priority: 27.03.78 US 890101

(43) Date of publication of application:
03.10.79 Bulletin 79/20

(45) Publication of the grant of the European patent:
23.12.81 Bulletin 81/51

(84) Designated Contracting States:
BE CH DE FR GB IT NL SE

(56) References cited:

US - A - 2 601 287

JOURNAL OF THE AMERICAN CHEMICAL SOCIETY, Vol. 72, June 1950, Washington DC (USA)
F. W. Heyl et al.: "Progresterone from 3-avetoxybisnor-5-cholenaldehyde and 3-ketobisnor-4-cholenaldehyde", pages 2617—19.

(73) Proprietor: HENKEL CORPORATION
4620 West 77th Street
Minneapolis Minnesota 55435 (US)

(72) Inventor: Krbechek, LeRoy O.
2041 Orkla Drive
Golden Valley Minnesota 55427 (US)

(74) Representative: Watkins, Arnold Jack et al,
European Patent Attorney Frank B. Dehn & Co.
Imperial House 15-19 Kingsway
London WC2B 6UZ (GB)

EP 0 004 430 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# A process for the selective conversion of 20-methylpregna-3,5,20(21)-triene-3,21-diol-diacetate to 3-oxo-20-methyl-pregna-4,20(21)-diene-21-yl-acetate

The present invention relates to a process for the selective conversion of 20-methylpregna-3,5,20(21)-triene-3,21-diol-diacetate to 3-oxo-20-methylpregna-4,20(21)-diene-2l-yl-acetate the process providing improved recovery of the latter compound which is a useful intermediate in the preparation of sex hormones.

It has long been known that many sex hormones may be prepared chemically through a variety of oxidation and reduction reactions. For instance, in an article entitled "Progesterone from 3-acetoxy-bisnor-5-cholenaldehyde and 3-ketobisnor-4-cholenaldehyde" by Heyl et al, June 1950, JACS; pp. 2617-2619, the aforementioned compounds are described as useful in the production of progesterone.

Basically the Heyl et al article, supra, discloses that the above compounds may be treated with acetic anhydride using sodium acetate as a catalyst to form the corresponding bisenol acetate and the 21 enol acetate. The use of the terms enol acetate and bisenol acetate herein correspond to 3-oxo-20-methylpregna-4,20(21)-diene-2l-yl-acetate and 20-methylpregna-3,5,20(21)-triene-3,21-diol-diacetate respectively. It is also noted that 3-keto-bisnor-4-cholenaldehyde referred to in Heyl et al is also known as 3-keto-4-pregnene-20-carboxaldehyde. The enol and bisenol compounds are then treated as discussed in the reference to give progesterone by chemical conversion.

In particular, one aspect of the heyl et al reference disclosed that by using stigmasterol as a starting material in an Oppenauer oxidation stigmastadienone is obtained. By ozonolysis of the stigmastadienone a relatively high yield of 3-ketobisnor-4-cholenaldehyde may be obtained. The 3-ketobisnor-4-cholenaldehyde is then converted to the abovementioned enol acetate and bisenol acetate by heating at reflux under nitrogen for six hours in a mixture of acetic anhydride and sodium acetate. The product of the acetic anhydride treatment which is described as a slightly yellow residue, is then dissolved in chloroform. The insoluble sodium acetate remaining in the reaction mixture may be filtered off and washed with additional chloroform to increase the recovery of the enol acetate and bisenol acetate products.

The enol and bisenol acetates are then described as being subjected to ozonolysis, followed by vacuum removal of the solvent. This residue comprising the ozonized product is then taken up in acetic acid and ether followed by mixing with zinc dust. This mixture is then diluted with ether, filtered, and the ether solution washed with a sodium hydroxide solution, water and dried. The ether is then evaporated and the residue is refluxed with a mixture of methanol and sulfuric acid to hydrolize and C-3 enol ester which may be present in the ozonized mixture. The Heyl et al reference then goes on further to state that this latter solution may be concentrated by vacuum to half its volume and extracted with ether. The ether solution is then stated to be washed with sodium hydroxide, water and dried over sodium sulfate. Progesterone is stated to be obtained by taking the dried residue up in anhydrous ether through warming and then allowing the prisms of progesterone to separate on standing. The over-all yield of progesterone is stated to be 60%.

It has now been found that by following the process described in the Heyl et al reference, by-products are obtained which diminish the potential yield of progesterone. Moreover, these by-products are extremely difficult to separate from progesterone due to the similar structure. The by-products comprise a mixture of 6-$\alpha$ and 6-$\beta$ hydroxy progesterones. The production of these 6-hydroxy progesterone compounds has been found to be proportional to the amount of bisenol acetate present in the mixture of enol acetate and bisenol acetate.

It is therefore desirable to minimize the amount of the bisenol acetate which is present with the enol acetate in order to increase greatly the amount of progesterone formed upon subsequent processing.

According to the present invention we provide a process for the selective conversion of 20-methyl-pregna-3,5,20(21)-triene-3,21-diol-diacetate to 3-oxo-20-methyl-pregna-4,20(21)-diene-21-yl-acetate, which comprises treating 20-methylpregna-3,5,20(21)-triene-3,21-diol-diacetate with at least one halogen-containing acid in the presence of at least one halogen-containing organic solvent.

The bisenol acetate is generally treated with a sufficient amount of the halogen-containing acid until conversion to the enol acetate is substantially complete.

Throughout the specification and claims, percentages and ratios are given by weight and temperatures are in degrees Celsius, unless otherwise indicated.

The present invention thus relates to a method which may be used to improve the yield of progesterone through the route described in the Heyl et al article. Using the method according to the present invention, it is possible to substantially increase the yield of progesterone over the procedure described in the reference. In carrying out the acetylation reaction described in the article to form the enol acetate and bisenol acetate, it has been found that the initial yield on the enol acetate is often about 70 to 80% based on the initial aldehyde. The yield of bisenol acetate in the mixture with the enol acetate is approximately 20 to 30% based on the starting aldehyde. The formulae shown below indicate the structure of (I) the starting aldehyde; (II) the bisenol acetate; and (III) the enol acetate; (IV) 6-$\alpha$/6-$\beta$ hydroxy progesterone; and (V) progesterone.

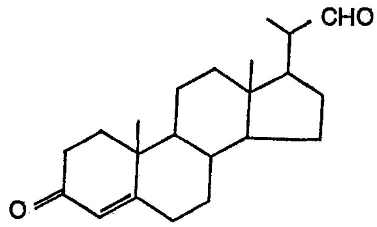

I

3-ketobisnor-4-cholenaldehyde also referred to as 3-keto-4-pregnene-20-carboxaldehyde)

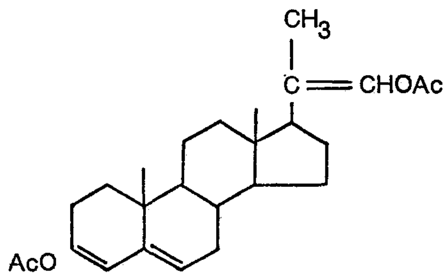

II

BISENOL ACETATE

(Where Ac is $CH_3\overset{\overset{O}{\|}}{C}$—)

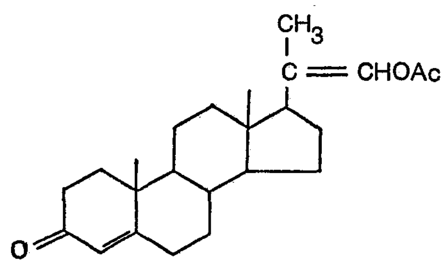

III

ENOL ACETATE

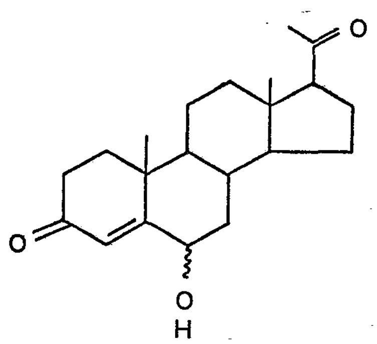

IV

6 alpha (beta) hydroxy progesterone

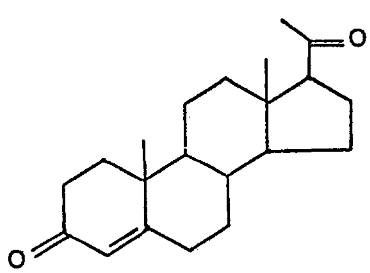

V

PROGESTERONE

In carrying out the selective conversion of the bisenol acetate to the enol acetate it has been found that serious problems are encountered. For example, it is first necessary to ensure that only the 3-acetoxy group is deacetylated leaving the 21-acetoxy group intact.

The foregoing point on not disturbing the 21-acetoxy structure is important since a preferred aspect of the present invention involves treating a mixture of the enol acetate and the bisenol acetate

without first separating the compounds. On the other hand, the method according to the invention may, if desired, be utilized to convert substantially pure bisenol acetate to the enol acetate. Where a mixture of the bisenol acetate and the enol acetate is used the respective weight ratio is conveniently from 15:1 to 1:40, preferably from 5:1 to 1:30.

It has first been found that halogen-containing acids of considerable acid strength may be used to accomplish the deacetylation. Other strong acids such as oxalic acid and para-toluene-sulfonic acid when used in place of the halogen-containing acids did not result in substantial conversion to the desired enol acetate.

Secondly, in view of the fact that a strong proton donating acid is needed to convert the bisenol acetate to the enol acetate, it is necessary to select a suitable solvent system for accomplishing this organic reaction. As mentioned above, oxalic and para-toluenesulfonic acids which are extremely strong proton donors have been found to be unsuitable in the 3-acetoxy deacetylation reaction when compared to the halogen-containing acids. It has been found that mixtures of the enol acetate and bisenol acetate are substantially insoluble in water. While such mixtures could be diluted to infinity in an aqueous solution followed by 3-acetoxy deacetylation, this would not be economical considering the amount of water which must be eventually removed from the reaction mixture in order to concentrate the desired end product.

Thus, in the present invention, it has been found first that deacetylation of the 3-acetoxy group of the bisenol acetate may be effected by a halogen-containing acid; and secondly, that by using a halogen-containing organic solvent the reaction proceeds quite rapidly with almost total conversion to the desired enol acetate.

Suitable solvents for use in the present invention include practically any available halogen-containing organic solvent. Preferably, however, the halogen-containing solvent will be a liquid at the operating temperatures of the reaction to avoid undue use of pressurizing equipment. The degree of halogen content of the solvent is not particularly critical. It has been found, however, that the preferred solvents for use in converting the bisenol acetate to the enol acetate are chlorine or bromine-containing solvents, such as methylene chloride, chloroform, ethylene dichloride, methylene bromide, 2-chloroethanol and carbon tetrachloride. The preferred solvent for conducting the reaction of the present invention is methylene chloride.

If desired, the solvent may comprise a mixture of halogen-containing solvents. A particularly preferred solvent system is 2-chloroethanol in admixture with methylene chloride. Mixtures of the halogen-containing solvents (e.g. methylen chloride and 2-chloroethanol) are conveniently employed at from about 20:1 to about 1:2, more preferably about 15:1 to about 2:1 by volume.

With respect to the selection of the acid for the 3-acetoxy deacetylation, it is again noted that oxalic and para-toluenesulfonic acids were found to be unsuitable. This unsuitability of aqueous solutions of the foregoing acids is believed to be due to a lack of solubility in the organic system. It is also noted that some halogen-containign acids which may be used in the present invention are not preferred due to their ability to strongly oxidize as well as to deacetylate. Particularly preferred halogen-containing acids for use in the process according to the invention, include hydrochloric acid, halogen-containing acetic acids (such as trichloroacetic acid and trifluoroacetic acid), trifluoromethanesulfonic acid, hydrobromic acid, hydrofluoric acid and hydroiodic acid.

It is also noted that, for some unexplained reason, the location of the halogen entity within the halogen-containing acid is apparently unimportant to the success of the acid in the deacetylation reaction. for example, trichloroacetic acid where the chlorine atoms are covalently bonded to a carbon atom is usable in the process as is hydrochloric acid which presents the halogen as an ion.

Of the aforementioned acids, hydroiodic acid which may be used is perhaps the least suitable of the halogen-containing acids due to its oxidising properties. Concentrated aqueous hydrochloric acid is preferred owing to its high acid strength and suitable solubility in the halogen-containing solvents. A preferred combination of halogen-containing solvent and halogen-containing acid is methylene chloride and hydrochloric acid. The system may be further improved, if desired, by the addition as co-solvents of short chain aliphatic acids, such as acetic acid, preferably glacial acetic acid. Further improvements may be obtained by utilizing dioxan as a co-solvent with the halogen-containing solvent. The cosolvent is conveniently used in a volume ratio to the halogen-containing acid of from about 20:1 to about 1:2, preferably from about 15:1 to about 2:1.

For the process of the present invention it is generally desirable to use the halogen-containing acid in as concentrated a form as possible to maximize the 3-acetoxydeacetylation of the bisenol acetate. In general therefore the halogen-containing acid will advantageously be solvated by a quantity of water or an alcohol such as methanol, ethanol or isopropanol, sufficient to effect freeing of the proton in the halogen-containing solvent.

In general therefore, the typical methods of measuring the strength of the acid utilized in the process according to the present invention, are unsuitable as the reaction mixture will rarely have a substantial water content and as the concept of pH is more usually applicable to aqueous solutions. In an attempt to quantify the amount of hydrogen ions necessary to accomplish the deacetylation it is suggested that the weight ratio of the acidic hydrogen concentration of the halogen-containing acid to the halogen-containing solvent should be from $10^{-1}$:1 to $10^{-6}$:1. That is, the weight ratio represents

the available protons in the acid, which would be available if the acid were dissolved in water. This weight ratio of the acidic hydrogen concentration of the halogen-containing acid to the halogen-containing solvent is preferably from $10^{-3}$:1 to $10^{-5}$:1.

The halogen-containing solvent utilized in the deacetylation may be employed in any convenient amount preferably in a weight ratio to the bisenol acetate of from about 500:1 to about 1:1, most preferably from about 100:1 to about 2:1.

It has also been found that following the conversion of the bisenol acetate to the enol acetate in accordance with the present invention, an aqueous wash of the reaction mixture may provide still higher yields of progesterone following ozonolysis than if the halogen-containing acid is not so washed out. It has further been found that an alkaline wash with a material such as sodium hydroxide following the initial aqueous wash may give still greater progesterone conversion. Thus, while an alkaline wash may be used first even better results are obtained where the first aqueous wash is neutral. These results are unexplained but nonetheless beneficial in increasing the yield of progesterone.

The treatment in accordance with the present invention may be effected over a wide temperature range, conveniently from about −20° to about 50°, preferably from −5° to +10°C.

The following Example illustrates the present invention:

Example

A mixture of the bisenol acetate and the enol acetate is obtained having an analysis showing less than 0.1% of the starting aldehyde (3-ketobisnor-4-cholenaldehyde), 38% of the enol acetate and approximately 60% of the bisenol acetate.

The mixture of the bisenol acetate and the enol acetate weighing 480 grams is dissolved in 1.5 litres of methylene chloride and filtered free of inorganic salts. It is noted that potassium acetate is used as a catalyst in the reference procedure for manufacturing the enol acetate mixture thus accounting for the salt in the mixture. The inorganic residue is then washed with an additional quantity of about 0.5 liter methylene chloride such that the total methylene chloride filtrates equal about 2 liters.

The methylene chloride and the mixture of the bisenol acetate and enol acetate is maintained under constant stirring at 3°C for ten hours in the presence of 20 milliliters of concentrated hydrochloric acid (13.1 molar in water).

At the end of the ten hour period the mixture was successively washed with 1 liter of water, 1 liter of a 2% sodium hydroxide solution, and an additional 1 liter of water before drying over calcium sulfate. The calcium sulfate is then removed by filtration and washed with additional methylene chloride thereafter combining the filtrates. The methylene chloride is then removed at reduced pressure leaving 372 grams of solid residue. A gas chromatograph analysis of a sample of the residue shows 1.4% of the starting aldehyde and 85.1% of the enol acetate. No bisenol acetate was reported.

Substantially similar results may be obtained by substituting chloroform, ethylene dichloride, 2-chloroethanol, methylene bromide or carbon tetrachloride for the methylene chloride solvent. Moreover, similar conversion yields are observed upon substituting aqueous solutions of trichloroacetic acid, trifluoroacetic acid, trifluoromethanesulfonic acid, hydrobromic acid, hydrofluoric acid or hydroiodic acid, for the hydrochloric acid.

An overall increase in the rate of enol acetate formation is found by utilizing 2-chloroethanol or glacial acetic acid in a ten fold (V/V) excess over the hydrochloric acid in the above example.

Thus, the present invention accomplishes the goal of converting the bisenol acetate to the enol acetate thereby minimizing the presence of by-products which diminish the overall yield of progesterone.

## Claims

1. A process for the selective conversion of 20-methylpregna-3,5,20(21)-triene-3,21-diol-diacetate to 3-oxo-20-methylpregna-4,20(21)-diene-21-yl-acetate, which comprises treating 20-methylpregna-3,5,20(21)-triene-3,21-diol-diacetate with at least one halogen-containing acid in the presence of at least one halogen-containing organic solvent.

2. A process as claimed in claim 1 wherein 20-methylpregna-3,5,20(21)-triene-3,21-diol-diacetate is initially in admixture with 3-oxo-20-methylpregna-4,20(21)-diene-21-yl-acetate in weight ratio of from 15:1 to 1:40.

3. A process as claimed in either of claims 1 and 2 wherein the said halogen-containing acid is selected from hydrochloric acid, trichloroacetic acid, trifluoroacetic acid, trifluoromethanesulfonic acid, hydrobromic acid, hydrofluoric acid, and hydroiodic acid and mixtures thereof.

4. A process as claimed in any of the preceding claims,wherein the weight ratio of the acidic hydrogen concentration of the halogen-containing acid to the halogen-containing solvent is from $10^{-1}$:1 to $10^{-6}$:1.

5. A process as claimed in any of the preceding claims wherein the said halogen-containing solvent comprises a chlorine- or bromine-containing solvent.

6. A process as claimed in claim 5 wherein the said halogen-containing solvent is selected from

methylene chloride, chloroform, ethylene dichloride, methylene bromide, 2-chloroethanol and carbon tetrachloride and mixtures thereof.

7. A process as claimed in claim 6 wherein the said halogen-containing solvent comprises a mixture of methylene chloride and 2-chloroethanol.

8. A process as claimed in any of the preceding claims wherein the said halogen-containing acid comprises hydrochloric acid and the said halogen-containing solvent comprises methylene chloride.

9. A process as claimed in any of the preceding claims wherein the said halogen-containing solvent is employed in conjunction with a cosolvent comprising a short chain aliphatic acid.

10. A process as claimed in any of the preceding claims wherein the resulting reaction mixture is subjected to a neutral aqueous wash step and then to an alkaline wash step.

**Revendications**

1. Procédé pour la conversion sélective du diacétate de 20-méthylprégna-3,5-20(21)-triène-3,21 diol en acétate de 3-oxo-20-méthylprégna-4,20(21)-dién-21-yle, qui consiste à traiter le diacétate de 20-méthylprégna-3,5,20(21)-triène-3,21-diol par au moins un acide halogéné en présence d'au moins un solvant organique halogéné.

2. Procédé selon la revendication 1, dans lequel le diacétate de 20-méthylprégna-3,5,20(21)-triène-3,21-diol est initialement en mélange avec l'acétate de 3-oxo-20-méthylprégna-4,20(21)-diène-21-yle en un rapport de poids compris entre 15:1 et 1:40.

3. Procédé selon l'une des revendications 1 et 2, dans lequel l'acide halogéné est choisi entre l'acide chlorhydrique, l'acide trichloracétique, l'acide trifluoracétique, l'acide trifluorométhane-sulfonique, l'acide bromhydrique, l'acide fluorhydrique et l'acide iodhydrique et leurs mélanges.

4. Procédé selon l'une quelconque des revendications précédentes, dans lequel le rapport de poids entre la concentration d'hydrogène acide de l'acide halogéné et le solvant halogéné est compris entre $10^{-1}$:1 et $10^{-6}$:1.

5. Procédé selon l'une quelconque des revendications précédentes, dans lequel le solvant halogéné comprend un solvant chloré ou bromé.

6. Procédé selon la revendication 5, dans lequel le solvant halogéné est choisi entre le chlorure de méthylène, le chloroforme, le dichlorure d'éthylène, le bromure de méthylène, le 2-chloréthanol et le tétrachlorure de carbone et leurs mélanges.

7. Procédé selon la revendication 6, dans lequel le solvant halogéné comprend un mélange de chlorure de méthylène et de 2-chloréthanol.

8. Procédé selon l'une quelconque des revendications précédentes, dans lequel l'acide halogéné est l'acide chlorhydrique et le solvant halogéné est le chlorure de méthylène.

9. Procédé selon l'une quelconque des revendications précédentes, dans lequel on utilise le solvant halogéné conjointement avec un cosolvant comprenant un acide aliphatique à chaîne courte.

10. Procédé selon l'une quelconque des revendications précédentes, dans lequel on soumet le mélange réactionnel obtenu à une étape de lavage aqueux neutre puis à une étape de lavage alcalin.

**Patentansprüche**

1. Ein Verfahren zur selektiven Umwandlung von 20-Methylpregna-3,5,20(21)-trien-3,21-diol-diacetat in 3-Oxo-20-methylpregna-4,20(21)-dien-21-yl-acetat, welches die Behandlung von 20-Methylpregna-3,5,20(21)-trien-3,21-diol-diacetat mit mindestens einer halogenhaltigen Säure in Gegenwart mindestens eines halogenhaltigen organischen Lösungsmittels umfaßt.

2. Ein Verfahren wie in Anspruch 1 beansprucht, worin 20-Methylpregna-3,5,20(21)-trien-3,21-diol-diacetat anfänglich im Gemisch mit 3-Oxo-20-methylpregna-4,20(21)-dien-21-yl-acetat im Gewichtsverhältnis von 15:1 bis 1:40 vorliegt.

3. Ein Verfahren wie in einem der Ansprüche 1 und 2 beansprucht, worin die halogenhaltige Säure aus Chlorwasserstoffsäure, Trichloressigsäure, Trifluoressigsäure, Trifluormethansulfonsäure, Bromwasserstoffsäure, Fluorwasserstoffsäure und Iodwasserstoffsäure und Gemischen daraus ausgewählt ist.

4. Ein Verfahren wie in einem der vorhergehenden Ansprüche beansprucht, worin das Gewichtsverhältnis der sauren Wasserstoffkonzentration der halogenhaltigen Säure zu dem halogenhaltigen Lösungsmittel von $10^{-1}$:1 bis $10^{-6}$:1 beträgt.

5. Ein Verfahren wie in einem der vorhergehenden Ansprüche beansprucht, worin das halogenhaltige Lösungsmittel ein chlor- oder bromhaltiges Lösungsmittel umfaßt.

6. Ein Verfahren wie in Anspruch 5 beansprucht, worin das halogenhaltige Lösungsmittel aus Methylenchlorid, Chloroform, Ethylendichlorid, Methylenbromid, 2-Chlorethanol und Tetrachlorkohlenstoff und Gemischen daraus ausgewählt ist.

7. Ein Verfahren wie in Anspruch 6 beansprucht, worin das halogenhaltige Lösungsmittel aus einem Gemisch aus Methylenchlorid und 2-Chlorethanol besteht.

8. Ein Verfahren wie in einem der vorhergehenden Ansprüche beansprucht, worin die halogenhaltige Säure aus Chlorwasserstoffsäure besteht und das halogenhaltige Lösungsmittel aus Methylenchlorid besteht.

9. Ein Verfahren wie in einem der vorhergehenden Ansprüche beansprucht, worin das halogenhaltige Lösungsmittel zusammen mit einem eine kurzkettige aliphatische Säure umfassenden Verschnittmittel verwendet wird.

10. Ein Verfahren wie in einem der vorhergehenden Ansprüche beansprucht, worin das erhaltene Reaktionsgemisch einer neutralen wäßrigen Waschstufe und dann einer alkalischen Waschstufe unterworfen wird.